# EUROPEAN PATENT SPECIFICATION

(11) **EP 3 025 643 B1**
(45) Date of publication and mention of the grant of the patent: **29.11.2017**
(21) Application number: 16150456.8
(22) Date of filing: 19.04.2007
(51) Int. Cl.: A61B 5/00

(54) **METHOD TO DETERMINE VISCERAL ADIPOSE TISSUE IN INFANTS**
METHODE ZUR BESTIMMUNG DES VISZERALEN FETTGEWEBES IN KLEINKINDERN
METHODE POUR QUANTIFIER LE TISSUE VISCERAL ADIPEUX CHEZ L'ENFANT

(30) Priority: 02.11.2006 WO PCT/NL2006/050274
(43) Date of publication of application: 01.06.2016
(62) Divisional of application: 07747386.6
(73) Proprietor: N.V. Nutricia, 2712 HM Zoetermeer (NL)
(72) Inventor: Zwijsen, Renate Maria Louise, 3734 GK Den Dolder (NL); van der Beek, Eline Marleen, 3584 CT Utrecht (NL); Speelmans, Gelske, 3584 CT Utrecht (NL); Boehm, Günther, 04107 Leipzig (DE)
(74) Representative: Nederlandsch Octrooibureau

(56) References cited:
- SUZUKI R ET AL: "Abdominal wall fat index, estimated by ultrasonography, for assessment of the ratio of visceral fat to subcutaneous fat in the abdomen", AMERICAN JOURNAL OF MEDICINE, EXCERPTA MEDICA, INC, UNITED STATES, vol. 95, no. 3, 1 September 1993 (1993-09-01), pages 309-314, XP023306348, ISSN: 0002-9343, DOI: 10.1016/0002-9343(93)90284-V [retrieved on 1993-09-01]
- HARRINGTON T A M ET AL: "Fast and reproducible method for the direct quantitation of adipose tissue in newborn infants", LIPIDS 2002 AMERICAN OIL CHEMISTS SOCIETY US, vol. 37, no. 1, 2002, pages 95-100, XP002755903,
- TAYLOR R W ET AL: "Evaluation of waist circumference, waist-to-hip ratio, and the conicity index as screening tools for high trunk fat mass, as measured by dual-energy X-ray absorptiometry, in children aged 3-19 y.", THE AMERICAN JOURNAL OF CLINICAL NUTRITION AUG 2000, vol. 72, no. 2, August 2000 (2000-08), pages 490-495, XP55261359, ISSN: 0002-9165
- STOLK R P ET AL: "Validity and reproducibility of ultrasonography for the measurement of intra-abdominal adipose tissue", INTERNATIONAL JOURNAL OF OBESITY, NEWMAN PUBLISHING, LONDON, GB, vol. 25, no. 9, 1 September 2001 (2001-09-01), pages 1346-1351, XP002519006, ISSN: 0307-0565, DOI: 10.1038/SJ.IJO.0801734

## Description

### FIELD OF THE INVENTION

The present invention relates to a method to determine visceral adipose tissue and/or the ratio of visceral to subcutaneous fat tissue in human subjects below 48 months of age.

### BACKGROUND OF THE INVENTION

Breast-feeding is the preferred method of feeding infants. Breast feeding early in life may influence the occurrence of disorders later in life. However, there are circumstances that make breast-feeding impossible or less desirable. In those cases infant formula and follow-on formula are a good alternative. The composition of modern infant or follow-on formulas is adapted in such a way that it meets many of the special nutritional requirements of the fast growing and developing infant.

Still it seems that improvements can be made towards the constitution of infant milk formula. For example little is known about the effects of ingredients in the infant formula on visceral adiposity and/or health later in life. The present invention relates to such future health.

WO 2006069918 describes a method of continuously reducing the circulating level of insulin like growth factor 1 (IGF-1) in the first few months of the life of an infant by administering to the infant a nutritional composition comprising proteins in an amount such that the composition contains less than 2.25g of protein per 100kcal. As IGF-1 is known to be a key control point in nutritional regulation of growth, this may offer a method of reducing the risk of developing obesity later life. WO 2007004878 relates to a method to treat and/or prevent childhood obesity comprising administering a nutritional composition containing fat, digestible carbohydrates and protein, wherein the protein comprises at least 25 wt.% peptides with a chain length of 25 to 30 amino acids based on dry weight of protein.

### SUMMARY OF THE INVENTION

The present inventors have recognized that the total adipose tissue of infants is not a good predictor to determine the risks of diseases later in life. Body fat can be distributed and stored in fat tissue at different locations within the body. Different fat tissues have different metabolic effects, particularly in infants. Subcutaneous fat tissue in infants has the important function to maintain an adequate body temperature. Fat tissue deposited in the central part of the body (visceral fat) serves only as storage of energy. Furthermore, adipose cells at different locations differ in size and in their protein secretion profile that are potential regulators of glucose and lipid homeostasis. Importantly, visceral fat tissue is a highly metabolically active tissue that releases free fatty acids directly into the hepatic portal vein. The obtained free fatty acid fluxes have an impact on glucose metabolism and insulin sensitivity of the liver and subsequently lead to metabolic disorders. Therefore, a main contributor of the development of certain disorders later in life is the distribution of fat tissue, particularly in case of an imbalanced ratio between visceral and subcutaneous fat tissue and/or excessive visceral fat tissue development in early infancy. Visceral fat tissue accumulation, and in particularly an increased ratio of visceral fat/subcutaneous fat tissue, was found to be a main contributor of development disorders, particularly of metabolic and cardiovascular disorders, independent of overall obesity.

For adipose cell proliferation two critical windows have been determined: during late gestation/early infancy and to a lesser extend during puberty. Outside these time frames cell numbers remain approximately the same and persist throughout adulthood. Hence, it is highly desirable to direct in early infancy the distribution of body fat towards a relative low number of visceral fat cells compared to the number of subcutaneous fat cells. A relative low visceral adipocyte count prevents large amounts of visceral adipose tissue and subsequently prevents metabolic disorders later in life.

So far, research on factors influencing body fat distribution in early life has been hampered by a lack of safe and easy accessible techniques to measure visceral adipose tissue in infants. Therefore, the inventors developed a reliable method to determine by ultrasonography visceral and subcutaneous fat tissue distribution in children up to 4 years. This method is described in claim 1.

### DETAILED DESCRIPTION OF THE INVENTION

### Visceral adiposity

Visceral adiposity differs from general adiposity or general obesity. Human subjects may suffer from general obesity due to increased subcutaneous fat deposits, but not suffer from visceral adiposity. On the other hand, human subjects may suffer from visceral adiposity, without suffering from general obesity. The increased risk of health problems later in life, such as diabetes and cardiovascular diseases, is related to the occurrence of visceral adiposity and not to general obesity.
The term 'visceral adiposity' refers to a condition with increased visceral fat tissue. Visceral adiposity is typically caused by (accumulation of) excessive visceral fat tissue. Visceral fat, also known as organ fat, intra-abdominal fat, peritoneal fat or central fat, is normally located inside the peritoneal cavity as opposed to subcutaneous fat which is found underneath the skin and intramuscular fat which is found interspersed in skeletal muscles. Visceral fat includes the abdominal fat surrounding the vital organs and includes mesenteric fat, perirenal fat, retroperitoneal fat and preperitoneal fat (fat surrounding the liver). In humans beyond infancy, visceral adiposity is also referred to as central obesity, 'apple-shaped' obesity, 'android' obesity, 'abdominal' obesity, 'male-type' obesity, 'waist-predominant' obesity, 'truncal' obesity or 'masculine' obesity. A waist circumference above 102 cm in adult man or above 88 cm in adult women indicates the presence of visceral adiposity. Also a waist-hip ratio can be used as indicator for visceral adiposity. Hip-waist ratio's exceeding 0.9 in man and 0.85 in women indicate visceral adiposity. For children of 3-19 years old appropriate cutoffs for age- and sex-dependent waist circumferences can be found in Taylor et al, 2000 Am J Clin Nutr 72:490-495. Visceral fat stores in adults and children can be investigated by imaging techniques including computed tomography (CT), and magnetic resonance imaging (MRI). These methods are accurate imaging techniques for children below 4 years but the disadvantages are costs, radiation exposure (for CT) and the use is very limited to a research setting. Ultrasonography (US) has been proposed as an alternative non-invasive technique to measure subcutaneous and visceral fat stores, because it overcomes these disadvantages. However, for children up to 4 years so far no suitable ultrasound imaging techniques are available.
An adult is considered to suffer from visceral adiposity or to have accumulated visceral fat tissue to an excessive amount when at the umbilicus level the visceral adipose tissue (VAT) exceeds 100 cm² in man, or 90 cm² in women (Saito et al, 1009, Int J Obes Suppl 3:S226) as determined with imaging techniques.

A subject suffers from visceral adiposity when it meets one or more of the above criteria (regarding VAT, waist circumference or waist-hip ratio thresholds). Accumulation of visceral fat tissue to an excessive amount relates to the accumulation of visceral fat tissue to a level at which visceral adiposity occurs and can be determined by the same methods as described above for visceral adiposity.

A non-obese human below 36 months of age has gender specific body mass index (BMI)-for age below the 95^{th} percentile, more preferably below the 85^{th} percentile. BMI is an anthropometric measure defined as weight in kilograms divided by the square of length in meters. Tables with gender specific BMI-for age are publicly available for instance at the US National Center for Health Statistics.

### Method to determine visceral adiposity in human subjects below 48 months of age

Besides the highly expensive, patient-unfriendly and time-consuming MRI and CT methods, at present there are no suitable methods to determine visceral adiposity in children below 4 years. As already mentioned, ultrasonography (US) methods to detect visceral fat versus subcutaneous fat are not suitable for infants and young children below 4 years. This is due to several reasons, such as that the measurement is not sensitive enough (the visceral fat layers in infants are very thin: less than 10% of the total body fat), and the different morphology of immature and developing organs. The most often used US method in adults has been described by Armellini F., Basic Life Sci. 60:75-7 and Stolk RP, 2001, Int J Obes Relat Metab Disord 25:1346-51. This is an indirect method of fat layers measuring the intra-abdominal fat (IAM) distance: the distance between the peritoneum and 1. the (dorsal side of) the aorta and/or 2. the spine. In this area, central fat layers have been found, although it also includes all tissue that is present in the abdomen (e.g. intestine). This method has been applied by the inventors to children of 9, 12, 24, and 36 months and 6 years old by placing the electronic calipers longitudinally at the height of the navel. It was found that in infants below 36 months, particularly below 24 months, and more particularly in the first 12 months, the outcome was influenced by many confounders, such as that the intestine can be filled with varying amounts of air, varying size of abdomen, varying stool properties, varying muscle contraction status. Therefore, this method does not lead to a realistic estimation of visceral fat and fat distribution and is not suitable for human subjects below 48 months.

An ultrasonography method for adults as described by Suzuki et al, 1993, Am J Med 95:309-14, was adapted in order to make this method suitable for human subjects below 48 months of age. In both the present method and the Suzuki method, scanning was longitudinally from the xiphoid process to the navel along the midline (linea alba).

After intensive research, the following adaptations were found to be essential: 1) adaptation relating to the precise location of measurement, and 2) adaptation relating to the unit of measurement.

Ad 1: In the method of Suzuki, 1993, the reference point of the preperitoneal fat layer was determined by the maximal thickness and the reference point of the subcutaneous fat layer was determined by the minimal thickness. In the method according to the present invention, using (very) young children, the optimal image to measure with regard to midline cut and parallelity of the fat layers was chosen. Therefore, as the apical reference point the point was taken where the slope of the different fat layers (i.e. the subcutaneous fat layer and the preperitoneal fat layer) changes and the different fat layers start to be situated in parallel to each other.

Ad 2: In the present method areas instead of distances were measured in order to increase sensitivity of the method. It was found that measurement of preperitoneal fat in children below 48 months of age as the area of fat tissue 1 cm or 2 cm along the midline, starting from the reference point in direction of the navel, gave more reliable results than measurement of the distance of the linea alba to the peritoneum on top of the liver as in the Suzuki method. More remarkably, in the very young children with an age below 24 months, measurement of the area between 1 cm and 2 cm along the midline gave even better results. It was also found that measurements of subcutaneous fat in children below 48 months of age as the area of fat tissue of 1 cm or 2 cm length along the midline, starting from the reference point in direction of the navel, gave more reliable results than measurement of distance of the inner surface of subcutaneous tissue to the linea alba as in the Suzuki method. Remarkably also here, in the very young children with an age below 24 months, measurement of the area between 1 cm and 2 cm along the midline gave even better results than measurement of distances.

Hence, the present invention relates to a method to determine visceral adipose tissue and/or the ratio of visceral to subcutaneous fat tissue in human subjects below 48 months of age, preferably below 36, more preferably below 24, said method comprising the steps of:
i) ultrasonographic measuring of fat tissue in human subjects abdomen, wherein the human subject is scanned longitudinally from the xiphoid process to the navel along the midline (linea alba),
ii) taking as the apical reference measure point the point where the slope of the different fat layers (i.e. the subcutaneous fat layer and the preperitoneal fat layer) changes and the different fat layers start to be situated in parallel to each other, optionally taking the most optimal image to measure this starting point,
iii) measuring the area of preperitoneal fat of 1 cm and/or 2 cm length along the midline, starting from the reference point as described in step 2 in direction of the navel and/or measuring the area of preperitoneal fat between 1 cm and 2 cm along the midline, starting from the reference point as described in step 2 in direction of the navel,
iv) optionally measuring the area subcutaneous fat of 1 cm and/or 2 cm length along the midline starting from the reference point as described in step 2 in direction of the navel, and/or measuring the area of subcutaneous fat between 1 cm and 2 cm along the midline, starting from the reference point as described in step 2 in direction of the navel, and
wherein in case of determining the ratio of visceral to subcutaneous fat tissue, the ratio of preperitoneal fat and subcutaneous fat is calculated.

### Example 1: Low protein beneficially affects insulin sensitivity

*Protein preparations:* Feeding 1a comprised 1.7 g protein per 100 ml and 9.6 g lactose per 100 ml. The protein was composed of 40 wt.% casein and 60 wt.% whey protein. Feeding 1b comprised 1.2 g protein per 100 ml at a ratio of 40 wt.% hydrolyzed casein and 60 wt.% hydrolyzed whey and 9.6 g lactose per 100 ml. Feeding 2a and 2b were volume matched compositions of human milk and infant formula (IMF). Feeding 2a, human milk, comprised per 100 ml 65 kcal, 0.95 g protein, 6.46 g carbohydrate and 3.75 g lipid. Feeding 2b, IMF, comprised per 100 ml 68 kcal, 1.4 g protein, 7.3 g carbohydrate and 3.5 g fat

Feeding 3a and 3b were energy matched compositions of human milk and IMF. Feeding 3a, comprised per 100 ml 72 kcal, 1.17 g protein, 8.35 g carbohydrate and 3.60 g fat. Feeding 3b equals feeding 2b.

*Methods:* In total 26 adult male Wistar rats (aged 10 weeks at the start of the experiment) were housed individually. After a 4 h fasting period, 8-10 animals were fed 2 ml of a test composition. Subsequently, blood samples (200 µl) were collected in heparinised chilled tubes at t=0, 5, 10, 15, 30, 60, 90, and 120 minutes after feeding. The experiment was repeated (cross over design) after ate least 1 week rest. Plasma insulin was measured by radioimmunoassay (RIA, of Linco Research) according to the manufacturer's instructions. Plasma glucose was measured with an oxidase-peroxidase method in 96-wells format (Roche Diagnostics, #1448668). In one experiment feeding 1a was compared with feeding 1b. In two other similar experiments feeding 2a was compared to feeding 2b and feeding 3a was compared to feeding 3b, respectively.

*Results:* The post-prandial peak of glucose as well as of insulin was lower in rats fed low protein than in rats fed a meal with a higher protein content. The area under the curve (AUC) of insulin and, to a lesser extent, glucose is lower in rats fed lower protein (Table 1). Decreased levels of both insulin and glucose is indicative for a decreased insulin resistance and/or insensitivity, which is believed contribute to the prevention of central obesity later-in-life. The decreased insulin resistance (or increased insulin sensitivity) was confirmed by the Belfiori formula.

At the same time the AUC of the total amino acids was not significantly lower in rats consuming low protein feedings and the AUC of the essential amino acids (sum of threonine, histidine, lysine, phenylalanine, arginine, tryptophane, leucine, valine, isoleucine and methionine was comparable between feedings tested (data not shown) indicating a sufficient bioavailability of the proteins to ensure proper growth and development.

**Table 1: Effects of proteins on post-prandial peak time, maximal peak height and area under the curve of glucose and insulin.**

| Effect | Feeding 1a | Feeding 1b |
|---|---|---|
| Maximal peak height | | |
| Glucose(mMl ± se) | 10.50±0.67 | 9.92±0.35# |
| Insulin (pM/l ± se) | 491.9±107.6 | 359.9±48.1¶ |

| Sum 15 (± se) | | |
|---|---|---|
| Glucose (mM/l*15min) | 36.7±1.98 | 35.4±1.05 |
| Insulin (nM/l*15min) | 7.34±1.47 | 6.15±0.67* |

| Sum 30 (± se) | | |
|---|---|---|
| Glucose (mM/l*30min) | 44.7±1.9 | 43.8±1.3# |
| Insulin (nM/l*30min) | 13.8±1.3 | 12.2±1.3* |

| Sum 120 (± se) | | |
|---|---|---|
| Glucose (mM/l*120min) | 69.1±2.4 | 12.09+0.39¶ |
| Insulin (nM/l*120min) | 13.8±1.3 | 12.2±1.3* |
| Belfiore | 1.0157±0.0022 | 1.0191+0.0030* |

| | | |
|---|---|---|
| *: P=0.1 ¶: P≤0.15 #:P=<0.2 | | |

**Table 2: Effects of protein concentration on post-prandial peak time, maximal peak height and area under the curve of glucose and insulin.**

| Feeding | Volume matched | | Energy matched | |
|---|---|---|---|---|
| Effect | 2a | 2b | 3a | 3b |
| Maximal peak height | | | | |
| Glucose(mM ± se) | 6.18±0.13 | 6.69±0.21 | 6.80±0.25 | 6.34±0.12 |
| Insulin (nM ± se) | 1.52±0.13 | 2.03±0.19 | 0.77±0.07 | 1.17±0.12 |

| iAUC 15 (± se) | | | | |
|---|---|---|---|---|
| Glucose (mM* 15min) | 11.9±2.5 | 8.9±3.4 | 7.0±1.5 | 5.8±2.1 |
| Insulin (nM*15min) | 14.5±1.2 | 17.9±1.5# | 4.9±0.7 | 7.5±0.6# |

| iAUC 30 (± se) | | | | |
|---|---|---|---|---|
| Glucose (mM*30min)20.2±4.8 | | 11.7±5.4 | 12.4±2.9 | 9.3±3.9 |
| Insulin (nM*30min) | 24.3±2.3 | 33.3±2.6¥ | 7.6±0.9 | 9.9±0.9* |

| iAUC 120 (± se) | | | | |
|---|---|---|---|---|
| Glucose (mM*120min) | 81.7±20.8 | 53.0±26.6* | 41.2±12.9 | 41.8±24¶ |
| Insulin (nM*120min) | 37.3±5.3 | 55.9±8.0¥ | 15.1±1.8 | 16.0±1.5 |
| Belfiore | 1.0004 | 1.0003# | 1.0005 | 1.0004¶ |

| | | | | |
|---|---|---|---|---|
| #: P=0.02 ¥: P=0.05 *: P=0.1 ¶:P=0.2 | | | | |

### Example 2 Composition with lower protein concentration effects body fat distribution in infants as determined by ultrasound measurements

The effect of breastfeeding (low protein, between 0.6 to 1.0 g protein pr 100 ml) versus standard formula feeding (high protein, average of 1.4 g protein per 100 ml) on body fat and fat distribution children. 229 Dutch children were included that underwent an ultrasound examination of the abdomen at the average age of 13 months.

Body fat and fat distribution: All ultrasound examinations of this study were performed with an ATL HDI 5000 (WA, Bothell) with a linear (L 12-5) and a curved transducer (C7-4).
1 Preperitoneal and subcutaneous fat tissue was measured according to the method described by Suzuki et al (Am J Med 1993;95(3):309-14.), with the following adaptations: The change in slope of the different layers to parallel layers served as the apical reference point to measure. The optimal image to measure with regard to midline cut and parallelity of the fat layers was chosen. Preperitoneal fat was measured as areas of between 1cm and 2 cm length along the midline, starting from the reference point in direction of the navel (PP area). Subcutaneous fat was measured as areas between 1 cm and 2 cm length along the midline starting from the reference point in direction of the navel (SC area). Moreover, ratios of preperitoneal fat and subcutaneous fat were calculated. All measurements were performed off-line.
   Bottle feeding, feeding 2, was defined as duration of exclusively breastfeeding equal or smaller than 1 month versus breast feeding. Breast feeding, feeding 1, was defined as more than 4 months exclusively breast feeding. The infants were 13.1±2.2 and 13.1±2.3 months old in feeding group 1 and 2, respectively.
   All data were analyzed using ANOVA and the SPSS statistical package (SPSS Inc.®, Chicago, IL Version 11 for Windows).
2 Comparison of abdominal fat and fat distribution in breast fed versus bottle fed infants: After adjustment for maternal pre-pregnancy BMI and educational level, a clear trend toward lower ratio of preperitoneal fat / subcutaneous fat tissue was observed in feeding 1 having low protein levels. See Table 3. The body weights, body length and body mass index (BMI) were unchanged in both feeding groups. See Table 4. This is indicative for the finding that low protein levels in early nutrition can influence body fat composition and/or distribution in favour of less visceral fat tissue compared to the subcutaneous fat tissue.

Moreover, the described method of measurements of preperitoneal and subcutaneous fat tissue are a suitable method to investigate abdominal fat in groups of children below 48 months of age.

**Table 3: body fat distribution as a result of early nutrition with different protein levels**

| | Preperitoneal fat | Subcutaneous fat | PP/SC ratio | |
|---|---|---|---|---|
| Feeding 1 (n=71) | 0.10 | 0.25 | 0.43 | |
| Feeding 2 (n=65) | 0.11 | 0.23 | 0.56 | p=0.08 |

**Table4: Body characteristics of infants.**

| Characteristic child | | Feeding 1 (n=71) | Feeding 2 (n=65) | p value |
|---|---|---|---|---|
| SD score current height | | -0.04(±0.9) | +0.1(±0.9) | 0.31 |
| SD score current weight | | -0.35(±0.9) | -0.33(±0.8) | 0.89 |
| SD score current BMI | | -0.38(±1.0) | -0.49(±0.8) | 0.47 |

### Example 3: Blood glucose/insulin and non-digestible oligosaccharides

*Animals and treatment:* Adult male Wistar rats (n=7) were given a galacto-oligosaccharide (GOS) load, cellulose load or water via a gastric canula on day 1. A 6 ml bolus load was administered equal to 50% of their daily fiber intake; GOS was transgalacto-oligosaccharides obtained from Elix'or (Borculo Domo). Fiber was dissolved in water. About 24 h later (on day 2) an oral glucose tolerance test was carried out and the postprandial glucose and insulin course was monitored for 120 min upon the intragastric injection of a carbohydrate load (2 g/kg body weight). To this end blood samples were drawn repeatedly via a jugular vein canula. Intragastric injection of water or a cellulose solution in water on day 1 served as control. As the Elix'or preparation also comprised digestible carbohydrates (mainly lactose), the two control injections were co-administered with carbohydrates to correct for this.

*Results:* pre-treatment with GOS clearly decreased the amount of insulin secreted, resulting in significant (p<0.05) lower incremental AUC values. Blood glucose levels were not affected significantly. Pre-treatment with cellulose or water did not modulate the insulin secretion, see Table 5.

**Table 5: Insulin and glucose levels levels in rats.**

| Pre-treatment with: | AUC insulin (pM*30 min) | AUC glucose (mM*30 min) |
|---|---|---|
| Water | 41 ± 7 | 69 ± 10 |
| Cellulose | 46 ± 8 | 75 ± 9 |
| GOS | 22 ± 4 | 74 ± 15 |

### Example 4: Infant formula

Infant nutrition comprising a lipid component providing 48% of the total calories, a protein component providing 8% of the total calories and a digestible carbohydrate component providing 44% of the total calories; (i) the lipid component comprising based on total fatty acids: 10 wt.% LA; 20 wt.% MCFA; 0.2 wt.% DHA, 0.05 wt.% EPA; the LA/ALA ratio is 5.1; (ii) the digestible carbohydrate component comprising 51 gram lactose/100 gram powder; 0.36 g galacto-oligosaccharides with DP 2-6 and 0.4 g fructo-oligosaccharides with DP 7-60; (ii) the protein component comprising cow's milk protein. The label of the package of this infant nutrition indicates that it is used for preventing the development of one or more disorders later-in-life, wherein said disorder is selected from the group consisting of type 2 diabetes, fasting hyperglycaemia, insulin resistance, visceral adiposity, hyperinsulinemia, hypertension, cardiovascular disease, cerebrovascular disease, artherosclerose, dyslipidaemia, hyperuricaemia, fatty liver, osteoarthritis and sleep apnoea.

## Claims

1. A method to determine visceral adipose tissue and/or the ratio of visceral to subcutaneous fat tissue in human subjects below 48 months of age, said method comprising:
i) ultrasonographic measuring of fat tissue in the human subject's abdomen, wherein the human subject is scanned longitudinally from the xiphoid process to the navel along the midline (linea alba),
ii) taking as the apical reference measure point the point where the slope of the different fat layers, i.e. the subcutaneous fat layer and the preperitoneal fat layer, changes and the different fat layers start to be situated in parallel to each other,
iii) measuring the area of preperitoneal fat of 1 cm and/or 2 cm length along the midline, starting from the reference point as described in step 2 in direction of the navel and/or measuring the area of preperitoneal fat between 1 cm and 2 cm along the midline, starting from the reference point as described in step 2 in direction of the navel,
wherein in case of determining the ratio of visceral to subcutaneous fat tissue, the ratio of preperitoneal fat and subcutaneous fat is calculated.

2. The method according to claim 1, wherein in step ii) the most optimal image to measure said reference measure point is taken.

3. The method according to claim 1 or 2 further comprising
iv) measuring the area subcutaneous fat of 1 cm and/or 2 cm length along the midline starting from the reference point as described in step ii) in direction of the navel, and/or measuring the area of subcutaneous fat between 1 cm and 2 cm along the midline, starting from the reference point as described in step ii) in direction of the navel.

4. The method according to any one of claims 1-3, wherein the human subject is below 36 months of age.

5. The method according to any one of claims 1-4, wherein the human subject is below 24 months of age.

## Patentansprüche

1. Verfahren zur Bestimmung von viszeralem Fettgewebe und/oder dem Verhältnis von viszeralem zu subkutanem Fettgewebe in Menschen im Alter von weniger als 48 Monaten, wobei bei dem Verfahren
i) das Fettgewebe in dem Abdomen des Menschen mittels Ultraschall gemessen wird, wobei der Mensch längs vom Schwertfortsatz bis zum Bauchnabel entlang der Mittelinie (Linea alba) gescannt wird,
ii) als apikaler Referenzmesspunkt der Punkt genommen wird, an dem sich die Neigung der verschiedenen Fettschichten, d.h. der subkutanen Fettschicht und der präperitonealen Fettschicht, verändert und die verschiedenen Fettschichten beginnen, sich parallel zueinander zu befinden,
iii) die Fläche von präperitonealem Fett über 1 cm und/oder 2 cm Länge entlang der Mittellinie gemessen wird, wobei von dem Referenzpunkt, wie er in Schritt 2 beschrieben wurde, in Richtung des Bauchnabels begonnen wird, und/oder die Fläche von präperitonealem Fett zwischen 1 cm und 2 cm entlang der Mittellinie gemessen wird, wobei von dem Referenzpunkt, wie er in Schritt 2 beschrieben wurde, in Richtung des Bauchnabels begonnen wird,
wobei im Fall der Bestimmung des Verhältnisses von viszeralem zu subkutanem Fettgewebe das Verhältnis von präperitonealem Fett und subkutanem Fett berechnet wird.

2. Verfahren nach Anspruch 1, bei dem in Schritt ii) die bestmögliche Abbildung genommen wird, um den Referenzmesspunkt zu bestimmen.

3. Verfahren nach Anspruch 1 oder 2, bei dem ferner
iv) die Fläche von subkutanem Fett über 1 cm und/oder 2 cm Länge entlang der Mittellinie gemessen wird, wobei von dem Referenzpunkt, wie er in Schritt ii) beschrieben wird, in Richtung des Bauchnabels begonnen wird, und/oder die Fläche von subkutanem Fett zwischen 1 cm und 2 cm entlang der Mittellinie gemessen wird, wobei von dem Referenzpunkt, wie er in Schritt ii) beschrieben wird, in Richtung Bauchnabel begonnen wird.

4. Verfahren nach einem der Ansprüche 1 bis 3, bei dem der Mensch ein Alter von weniger als 36 Monaten aufweist.

5. Verfahren nach einem der Ansprüche 1 bis 4, bei dem der Mensch ein Alter von weniger als 24 Monaten aufweist.

## Revendications

1. Procédé pour déterminer le tissu viscéral adipeux et/ou le rapport du tissu graisseux viscéral à sous-cutané chez des sujets humains âges de moins de 48 mois, ledit procédé comprenant :
i) la mesure par ultrasonographie du tissu graisseux de l'abdomen du sujet humain, dans lequel le sujet humain est balayé longitudinalement de l'appendice xiphoïde à l'ombilic le long de la ligne médiane (ligne blanche),
ii) la considération, en tant que point de mesure de référence apical, du point où la pente des différent couches de graisse, à savoir la couche de graisse sous-cutanée et la couche de graisse prépéritonéale, change et les différentes couches de graisse commencent à être situées parallèlement les unes aux autres,
iii) la mesure de la surface de graisse prépéritonéale de 1 cm et/ou 2 cm de longueur le long de la ligne médiane, en commençant à partir du point de référence tel que décrit à l'étape 2 en direction de l'ombilic et/ou la mesure de la surface de graisse prépéritonéale entre 1 cm et 2 cm le long de la ligne médiane, en commençant à partir du point de référence tel que décrit à l'étape 2 en direction de l'ombilic,
dans lequel dans le cas d'une détermination du rapport du tissu graisseux viscéral à sous-cutané, le rapport de la graisse prépéritonéale et de la graisse sous-cutanée est calculé.

2. Procédé selon la revendication 1, dans lequel à l'étape ii) l'image la plus optimale pour mesurer ledit point de mesure de référence est prise.

3. Procédé selon la revendication 1 ou 2 comprenant en outre iv) la mesure de la surface de graisse sous-cutanée de 1 cm et/ou 2 cm de longueur le long de la ligne médiane en commençant à partir du point de référence tel que décrit à l'étape ii) en direction de l'ombilic, et/ou la mesure de la surface de graisse sous-cutanée entre 1 cm et 2 cm le long de la ligne médiane, en commençant à partir du point de référence tel que décrit à l'étape ii) en direction de l'ombilic.

4. Procédé selon l'une quelconque des revendications 1 à 3, dans lequel le sujet humain est âgé de moins de 36 mois.

5. Procédé selon l'une quelconque des revendications 1 à 4, dans lequel le sujet humain est âgé de moins de 24 mois.
